# EUROPEAN PATENT APPLICATION

(11) **EP 3 312 286 A2**
(43) Date of publication of application: **25.04.2018**
(21) Application number: 17194809.4
(22) Date of filing: 04.10.2017
(51) Int. Cl.: C12P 7/44

(54) **PROCESS FOR CONVERTING DINITRILES TO DICARBOXYLIC ACIDS**

(30) Priority: 05.10.2016 GB 201616934
(71) Applicant: Chemoxy International Limited, Middlesborough TS3 6AF (GB)
(72) Inventor: RANDALL, David, Middlesbrough, TS3 6AF (GB); BLACK, Gary, Newcastle-Upon-Tyne, NE1 8ST (GB)
(74) Representative: HGF Limited

(57) **Abstract**

The present invention relates to nitrilases and uses thereof, such as in a process for the production of a dicarboxylic acid comprising the step of:
i) reacting a composition comprising one or more aliphatic dinitriles wherein the or each aliphatic dinitrile contains from 2 to 20 carbon atoms or one or more aromatic dinitriles wherein the or each aromatic dinitrile contains from 6 to 20 carbon atoms with a nitrilase to produce a dicarboxylic acid, wherein the nitrilase comprises a CWE motif and, wherein the nitrilase when tested using the OPA Nitrilase Assay with 2-methylglutaronitrile as substrate has an % total hydrolysis of at least 45% or when tested using the OPA Nitrilase Assay with 4-CPA as substrate has an % total hydrolysis of at least 15%.

SEQUENCE LISTING

<110>  Chemoxy International Limited

<120>  Nitrile conversion process

<130>  P209368EP

<140>  EP 17194809.4
<141>  2017-10-04

<150>  GB 1616934.4
<151>  2016-10-05

<160>  8

<170>  PatentIn version 3.5

<210>  1
<211>  321
<212>  PRT
<213>  Bradyrhizobium diazoefficiens

<400>  1

Met Met Asp Ser Asn Arg Pro Asn Thr Tyr Lys Ala Ala Val Val Gln
1               5                   10                  15

Ala Ala Ser Asp Pro Thr Ser Ser Leu Val Ser Ala Gln Lys Ala Ala
            20                  25                  30

Ala Leu Ile Glu Lys Ala Ala Gly Ala Gly Ala Arg Leu Val Val Phe
        35                  40                  45

Pro Glu Ala Phe Ile Gly Gly Tyr Pro Lys Gly Asn Ser Phe Gly Ala
    50                  55                  60

Pro Val Gly Met Arg Lys Pro Glu Gly Arg Glu Ala Phe Arg Leu Tyr
65                  70                  75                  80

Trp Glu Ala Ala Ile Asp Leu Asp Gly Val Glu Val Glu Thr Ile Ala
                85                  90                  95

Ala Ala Ala Ala Ala Thr Gly Ala Phe Thr Val Ile Gly Cys Ile Glu
            100                 105                 110

Arg Glu Gln Gly Thr Leu Tyr Cys Thr Ala Leu Phe Phe Asp Gly Ala
        115                 120                 125

Arg Gly Leu Val Gly Lys His Arg Lys Leu Met Pro Thr Ala Gly Glu
    130                 135                 140

Arg Leu Ile Trp Gly Phe Gly Asp Gly Ser Thr Met Pro Val Phe Glu
145                 150                 155                 160

Thr Ser Leu Gly Asn Ile Gly Ala Val Ile Cys Trp Glu Asn Tyr Met
                165                 170                 175

Pro Met Leu Arg Met His Met Tyr Ser Gln Gly Ile Ser Ile Tyr Cys
            180                 185                 190

Ala Pro Thr Ala Asp Asp Arg Asp Thr Trp Leu Pro Thr Met Gln His
        195                 200                 205

Ile Ala Leu Glu Gly Arg Cys Phe Val Leu Thr Ala Cys Gln His Leu
    210                 215                 220

Lys Arg Gly Ala Phe Pro Ala Asp Tyr Glu Cys Ala Leu Gly Ala Asp
225                 230                 235                 240

Pro Glu Thr Val Leu Met Arg Gly Gly Ser Ala Ile Val Asn Pro Leu
                245                 250                 255

Gly Lys Val Leu Ala Gly Pro Cys Phe Glu Gly Glu Thr Ile Leu Tyr
            260                 265                 270

Ala Asp Ile Ala Leu Asp Glu Val Thr Arg Gly Lys Phe Asp Phe Asp
        275                 280                 285

Ala Ala Gly His Tyr Ser Arg Pro Asp Val Phe Gln Leu Val Val Asp
    290                 295                 300

Asp Arg Pro Lys Arg Ala Val Ser Thr Val Ser Ala Val Arg Ala Arg
305                 310                 315                 320

Asn

<210> 2
<211> 321
<212> PRT
<213> Artificial Sequence

<220>
<223> Variant nitrilase

<400> 2

Met Met Asp Ser Asn Arg Pro Asn Thr Tyr Lys Ala Ala Val Val Gln
1               5                   10                  15

Ala Ala Ser Asp Pro Thr Ser Ser Leu Val Ser Ala Gln Lys Ala Ala
            20                  25                  30

Ala Leu Ile Glu Lys Ala Ala Gly Ala Gly Ala Arg Leu Val Val Phe
        35                  40                  45

Pro Glu Ala Phe Ile Gly Gly Tyr Pro Lys Gly Asn Ser Phe Gly Ala
    50                  55                  60

Pro Val Gly Met Arg Lys Pro Glu Gly Arg Glu Ala Phe Arg Leu Tyr
65                  70                  75                  80

Trp Glu Ala Ala Ile Asp Leu Asp Gly Ala Glu Val Glu Thr Ile Ala
                85                  90                  95

Ala Ala Ala Ala Ala Thr Gly Ala Phe Thr Val Ile Gly Cys Ile Glu
            100                 105                 110

Arg Glu Gln Gly Thr Leu Tyr Cys Thr Ala Leu Phe Phe Asp Gly Ala
        115                 120                 125

Arg Gly Leu Val Gly Lys His Arg Lys Leu Met Pro Thr Ala Gly Glu
    130                 135                 140

Arg Leu Ile Trp Gly Phe Gly Asp Gly Ser Thr Met Pro Val Phe Glu
145                 150                 155                 160

Thr Ser Leu Gly Asn Ile Gly Ala Val Ile Cys Trp Glu Asn Tyr Met
                165                 170                 175

Pro Met Leu Arg Met His Met Tyr Ser Gln Gly Ile Ser Ile Tyr Cys
            180                 185                 190

Ala Pro Thr Ala Asp Asp Arg Asp Thr Trp Leu Pro Thr Met Gln His
        195                 200                 205

Ile Ala Leu Glu Gly Arg Cys Phe Val Leu Thr Ala Cys Gln His Leu
    210                 215                 220

Lys Arg Gly Ala Phe Pro Ala Asp Tyr Glu Cys Ala Leu Gly Ala Asp
225                 230                 235                 240

Pro Glu Thr Val Leu Met Arg Gly Gly Ser Ala Ile Val Asn Pro Leu
                245                 250                 255

Gly Lys Val Leu Ala Gly Pro Cys Phe Glu Gly Glu Thr Ile Leu Tyr
            260                 265                 270

Ala Asp Ile Ala Leu Asp Glu Val Thr Arg Gly Lys Phe Asp Phe Asp
        275                 280                 285

Ala Ala Gly His Tyr Ser Arg Pro Asp Val Phe Gln Leu Val Val Asp
    290                 295                 300

Asp Arg Pro Lys Arg Ala Val Ser Thr Val Ser Ala Val Arg Ala Arg
305                 310                 315                 320

Asn

<210>  3
<211>  321
<212>  PRT
<213>  Artificial Sequence

<220>
<223>  Variant nitrilase

<400>  3

Met Met Asp Ser Asn Arg Pro Asn Thr Tyr Lys Ala Ala Val Val Gln
1               5                   10                  15

Ala Ala Ser Asp Pro Thr Ser Ser Leu Val Ser Ala Gln Lys Ala Ala
            20                  25                  30

Ala Leu Ile Glu Lys Ala Ala Gly Ala Gly Ala Arg Leu Val Val Phe
        35                  40                  45

Pro Glu Ala Phe Ile Gly Gly Tyr Pro Lys Gly Asn Ser Phe Gly Ala
    50                  55                  60

Pro Val Gly Met Arg Lys Pro Glu Gly Arg Glu Ala Phe Arg Leu Tyr
65                  70                  75                  80

Trp Glu Ala Ala Ile Asp Leu Asp Gly Val Glu Val Glu Thr Ile Ala
                85                  90                  95

Ala Ala Ala Ala Ala Thr Gly Ala Phe Thr Val Ile Gly Cys Ile Glu
            100                 105                 110

Arg Glu Gln Gly Thr Leu Tyr Cys Thr Ala Leu Phe Phe Asp Gly Ala
        115                 120                 125

Arg Gly Leu Val Gly Lys His Arg Lys Leu Met Pro Thr Ala Gly Glu
    130                 135                 140

Arg Leu Ile Trp Gly Phe Gly Asp Gly Ser Thr Met Pro Val Phe Glu
145                 150                 155                 160

Thr Ser Leu Gly Asn Ile Gly Ala Val Ile Cys Trp Glu Asn Tyr Met
                165                 170                 175

Pro Met Leu Arg Met His Met Tyr Ser Gln Gly Ile Ser Ile Tyr Cys
            180                 185                 190

Ala Pro Thr Ala Asp Asp Arg Asp Thr Trp Leu Pro Thr Met Gln His
        195                 200                 205

Ile Ala Leu Glu Gly Arg Cys Phe Val Leu Thr Ala Cys Gln His Leu
    210                 215                 220

Lys Arg Gly Ala Phe Pro Ala Asp Tyr Glu Cys Ala Leu Gly Ala Asp
225                 230                 235                 240

Pro Glu Ala Val Leu Met Arg Gly Gly Ser Ala Ile Val Asn Pro Leu
                245                 250                 255

Gly Lys Val Leu Ala Gly Pro Cys Phe Glu Gly Glu Thr Ile Leu Tyr
            260                 265                 270

Ala Asp Ile Ala Leu Asp Glu Val Thr Arg Gly Lys Phe Asp Phe Asp
        275                 280                 285

Ala Ala Gly His Tyr Ser Arg Pro Asp Val Phe Gln Leu Val Val Asp
    290                 295                 300

Asp Arg Pro Lys Arg Ala Val Ser Thr Val Ser Ala Val Arg Ala Arg
305                 310                 315                 320

Asn

<210>  4
<211>  966
<212>  DNA
<213>  Bradyrhizobium elkanii CHANGE

<400>  4
atgatggata gcaaccgacc gaacacctac aaggcggccg tcgttcaggc cgcgtcagat      60

ccgacaagct cgctggtgtc ggcgcaaaag gcggcggccc tgatcgagaa ggccgctggc     120

gccggcgcga ggctggtcgt tttccctgaa gccttcattg gcggctatcc caagggaaat     180

tcgttcggcg ctcccgtcgg catgcgcaag cccgaaggac gcgaggcgtt ccgtttgtat     240

tgggaggcgg ccatcgatct cgatggcgtg gaggtcgaga cgatcgctgc ggcggccgcc     300

gcaaccggtg cctttaccgt catcggctgc atcgagcgag agcagggcac gctctactgc     360

acggcgctgt tcttcgacgg ggcgcgtggg ctcgtcggca agcaccgcaa gctgatgccg 420

actgcgggcg agcggctgat ctggggtttc ggcgacggct cgaccatgcc ggtgttcgag 480

acctcgctcg gcaatatcgg cgccgtgatc tgctgggaga actacatgcc gatgctgcgc 540

atgcacatgt acagccaggg catcagcatc tactgcgctc caaccgcgga tgatcgcgat 600

acctggcttc cgaccatgca gcatatcgcc ctggagggac gctgcttcgt cctgacggcc 660

tgtcagcatc tcaagcgcgg cgccttcccg gcggactacg agtgcgcgct gggtgccgat 720

cccgagaccg tgctcatgcg cggcggcagc gccatcgtca atccgctcgg caaggtgctg 780

gcgggtccat gcttcgaggg ggagaccatc ctgtatgccg acattgcgct cgacgaggtg 840

acgcgcggca agtttgattt cgacgccgcg gggcactatt cccgtccgga cgttttccag 900

ctcgtcgtcg acgatcgacc gaagcgggca gtatcaaccg tcagcgccgt tcgcgcgcgc 960

aattga 966

<210> 5
<211> 321
<212> PRT
<213> Artificial Sequence

<220>
<223> Variant nitrilase

<400> 5

Met Met Asp Ser Asn Arg Pro Asn Thr Tyr Lys Ala Ala Val Val Gln
1               5                   10                  15

Ala Ala Ser Asp Pro Thr Ser Ser Leu Val Ser Ala Gln Lys Ala Ala
            20                  25                  30

Ala Leu Ile Glu Lys Ala Ala Gly Ala Gly Ala Arg Leu Val Val Phe
        35                  40                  45

Pro Glu Ala Phe Ile Gly Gly Tyr Pro Lys Gly Asn Ser Phe Gly Ala
    50                  55                  60

Pro Val Gly Met Arg Lys Pro Glu Gly Arg Glu Ala Phe Arg Leu Tyr
65                  70                  75                  80

Trp Glu Ala Ala Ile Asp Leu Asp Gly Ala Glu Val Glu Thr Ile Ala
                85                  90                  95

Ala Ala Ala Ala Ala Thr Gly Ala Phe Thr Val Ile Gly Cys Ile Glu
            100                 105                 110

Arg Glu Gln Gly Thr Leu Tyr Cys Thr Ala Leu Phe Phe Asp Gly Ala
        115                 120                 125

Arg Gly Leu Val Gly Lys His Arg Lys Leu Met Pro Thr Ala Gly Glu
    130                 135                 140

Arg Leu Ile Trp Gly Phe Gly Asp Gly Ser Thr Met Pro Val Phe Glu
145                 150                 155                 160

Thr Ser Leu Gly Asn Ile Gly Ala Val Ile Cys Trp Glu Asn Tyr Met
                165                 170                 175

Pro Met Leu Arg Met His Met Tyr Ser Gln Gly Ile Ser Ile Tyr Cys
            180                 185                 190

Ala Pro Thr Ala Asp Asp Arg Asp Thr Trp Leu Pro Thr Met Gln His
        195                 200                 205

Ile Ala Leu Glu Gly Arg Cys Phe Val Leu Thr Ala Cys Gln His Leu
    210                 215                 220

Lys Arg Gly Ala Phe Pro Ala Asp Tyr Glu Cys Ala Leu Gly Ala Asp
225                 230                 235                 240

Pro Glu Thr Val Leu Met Arg Gly Gly Ser Ala Ile Val Asn Pro Leu
                245                 250                 255

Gly Lys Val Leu Ala Gly Pro Cys Phe Glu Gly Glu Thr Ile Leu Tyr
            260                 265                 270

Ala Asp Ile Ala Leu Asp Glu Val Thr Arg Gly Lys Phe Asp Phe Asp
        275                 280                 285

Ala Ala Gly His Tyr Ser Arg Pro Asp Val Phe Gln Leu Val Val Asp
    290                 295                 300

Asp Arg Pro Lys Arg Ala Val Ser Thr Val Ser Ala Val Arg Ala Arg
305                 310                 315                 320

Asn

<210>  6
<211>  966
<212>  DNA
<213>  Artificial Sequence

<220>
<223>  V90A nucleotide sequence

<400>  6

atgatggata gcaaccgacc gaacacctac aaggcggccg tcgttcaggc cgcgtcagat 60

ccgacaagct cgctggtgtc ggcgcaaaag gcggcggccc tgatcgagaa ggccgctggc 120

gccggcgcga ggctggtcgt tttccctgaa gccttcattg gcggctatcc caagggaaat 180

tcgttcggcg ctcccgtcgg catgcgcaag cccgaaggac gcgaggcgtt ccgtttgtat 240

tgggaggcgg ccatcgatct cgatggcgcg gaggtcgaga cgatcgctgc ggcggccgcc 300

gcaaccggtg cctttaccgt catcggctgc atcgagcgag agcagggcac gctctactgc 360

acggcgctgt tcttcgacgg ggcgcgtggg ctcgtcggca agcaccgcaa gctgatgccg 420

actgcgggcg agcggctgat ctggggtttc ggcgacggct cgaccatgcc ggtgttcgag 480

acctcgctcg gcaatatcgg cgccgtgatc tgctgggaga actacatgcc gatgctgcgc 540

atgcacatgt acagccaggg catcagcatc tactgcgctc caaccgcgga tgatcgcgat 600

acctggcttc cgaccatgca gcatatcgcc ctggagggac gctgcttcgt cctgacggcc 660

tgtcagcatc tcaagcgcgg cgccttcccg gcggactacg agtgcgcgct gggtgccgat 720

cccgagaccg tgctcatgcg cggcggcagc gccatcgtca atccgctcgg caaggtgctg 780

gcgggtccat gcttcgaggg ggagaccatc ctgtatgccg acattgcgct cgacgaggtg 840

acgcgcggca agtttgattt cgacgccgcg gggcactatt cccgtccgga cgttttccag 900

ctcgtcgtcg acgatcgacc gaagcgggca gtatcaaccg tcagcgccgt tcgcgcgcgc 960

aattga 966

<210> 7
<211> 68
<212> PRT
<213> Artificial Sequence

<220>
<223> Amidase

<220>
<221> misc_feature
<222> (4)..(5)
<223> Xaa can be any naturally occurring amino acid

<220>
<221> misc_feature
<222> (14)..(14)
<223> Xaa can be any naturally occurring amino acid

<220>
<221> misc_feature
<222> (16)..(17)
<223> Xaa can be any naturally occurring amino acid

<220>
<221> misc_feature
<222> (24)..(24)
<223> Xaa can be any naturally occurring amino acid

<220>
<221>  misc_feature
<222>  (34)..(34)
<223>  Xaa can be any naturally occurring amino acid

<220>
<221>  misc_feature
<222>  (36)..(36)
<223>  Xaa can be any naturally occurring amino acid

<220>
<221>  misc_feature
<222>  (40)..(40)
<223>  Xaa can be any naturally occurring amino acid

<220>
<221>  misc_feature
<222>  (45)..(45)
<223>  Xaa can be any naturally occurring amino acid

<220>
<221>  misc_feature
<222>  (48)..(48)
<223>  Xaa can be any naturally occurring amino acid

<220>
<221>  misc_feature
<222>  (51)..(51)
<223>  Xaa can be any naturally occurring amino acid

<220>
<221>  misc_feature
<222>  (53)..(53)
<223>  Xaa can be any naturally occurring amino acid

<220>
<221>  misc_feature
<222>  (56)..(57)
<223>  Xaa can be any naturally occurring amino acid

<220>
<221>  misc_feature
<222>  (65)..(65)
<223>  Xaa can be any naturally occurring amino acid

<220>
<221>  misc_feature
<222>  (67)..(67)
<223>  Xaa can be any naturally occurring amino acid

<400>  7

Met Ala Val Xaa Xaa Pro Thr Pro Thr Gln Leu Ala Ala Xaa Ala Xaa
1               5                   10                  15

Xaa Tyr Gly Leu Ser Leu Thr Xaa Glu Asp Val Ala Ser Phe Arg Pro
            20                  25                  30

Leu Xaa Gln Xaa Ser Leu Asp Xaa Tyr Asp Leu Val Xaa Glu Leu Xaa
        35                  40                  45

Asp Glu Xaa Pro Xaa Val Lys Xaa Xaa Arg Thr Pro Gly Tyr Arg Pro
    50                  55                  60

Xaa Ser Xaa Glu
65

<210>  8
<211>  4
<212>  PRT
<213>  Artificial Sequence

<220>
<223>  nitrilase motif

<400>  8

Cys Trp Glu Asn
1

## Description

The present invention relates to a process for preparing dicarboxylic acids from dinitriles in a biological process. Dicarboxylic acids have a wide range of uses including in the manufacture of synthetic fibres such as nylon and in the production of dibasic esters. More particularly, the process involves the production of dibasic acids from dinitriles including from alpha, omega dinitriles using a modified enzyme. The enzyme used in the process is a nitrilase and more specifically is a class 1 nitrilase (nitrile amino hydrolase, EC: 3.5.5.1).

The market for dibasic acids and for downstream products such as dibasic esters is large and growing. The annual global market for dibasic esters, for example, is about 100,000 tonnes per annum and the market value is about £150m. This market is growing at the rate of around 6% per year. At the same time, the key feedstock for preparing dibasic esters is the by-product of a mature and declining market.

Conventionally, nitriles are converted to carboxylic acids via an inorganic transformation in which the nitrile group is hydrolysed in the presence of acid or base to a carboxylic acid functionality. This process employs extensive amounts of sulphuric acid and sodium hydroxide and suffers the disadvantages of relatively high cost and the generation of substantial waste.

The existing inorganic chemistry approach used in the hydrolysis of nitriles to carboxylic acids involves the use of sodium hydroxide to convert the nitrile to the sodium salt of the carboxylic acid. This releases ammonia which is then trapped by sulphuric acid. The sodium salt of the carboxylic acid is then converted to the free acid by the treatment with further sulphuric acid. Overall, for each mole of nitrile functionality that is converted to a mole of carboxylic acid functionality, requires 1 mole of sulphuric acid and 1 mole of sodium hydroxide to be used.

The present invention overcomes this problem using a feedstock which is currently a waste stream from another industrial process, the process for preparing a precursor of nylon. Historically, this waste material has been disposed of by combustion.

The biotransformation route of the present invention avoids the need for the use of strong acids and bases and therefore avoids any issues with their disposal. In addition, not only is the biotransformation route of the invention more energy efficient in its own right, it also avoids the need for energy to be expended in producing reagents such as sodium hydroxide and sulphuric acid for use in a conventional process. In addition, the resulting products of the conventional process are sodium sulphate and ammonium sulphate. Sodium sulphate is effectively a waste product which does not have any industrial value. In contrast, a further benefit of the process of the invention is that in one embodiment the starting raw material, which is currently burnt for fuel value, and which has an impact on the environment by adding to the carbon dioxide burden in the atmosphere need not be burnt but instead the carbon can be reutilised in the synthesis of a valuable chemical product. The biotransformation process of the invention avoids the generation of carbon dioxide and will present a potentially valuable carbon offset value to industry in addition to reclaiming value from a waste resource.

One feedstock which can be used in the process of the present invention is the waste stream from the production of adiponitrile which is used in the manufacture of hexamethylenediamine (HMDA). HMDA is a key component in the production of nylon 66 fibres and resins. HMDA is combined with adipic acid in order to produce nylon 66. During the production of adiponitrile, significant amounts of 2-methyl glutaronitrile are produced along with smaller quantities of 2-ethyl succinonitrile. Historically, 2-methyl glutaronitrile has been burnt as it cannot be used in the preparation of nylon.

Currently, there is no commercial bio-catalytic process available for the production of dibasic carboxylic acids from dinitriles. Similarly, there are currently no known biocatalysts which are suitable for this process.

A number of nitrilases are known and these have been used in various bio-catalytic transformations to convert nitrile functionality to carboxylic acid functionality. Nitrile-hydrolysing enzymes frequently display enantioselectivity and regioselectivity which cannot normally be achieved via traditional inorganic transformations. There are two classes of nitrile hydrolysing enzymes, the first is nitrile hydratases (E.C. 4.2.1.84) which convert nitrile functionality to primary amides, and the second being nitrilases (E.C. 3.5.5.1) which convert nitriles to the related carboxylic acid. The present invention is concerned specifically with nitrilases.

The present invention differs from existing biotransformations of nitrile groups in that it does not use a wild type nitrilase. The nitrilase of the invention is a mutant enzyme. Suitable mutant enzymes can be determined from the assay which is described below.

In the case of a dinitrile, the nitrilase attacks both nitrile groups but, depending In the case of a dinitrile where one of the nitrile groups is more sterically hindered than the other, e.g. the chiral dinitrile 2-methyl glutaronitrile, a conventional nitrilase tends only to react at the unhindered nitrile group, in the case of 2-methyl glutaronitrile, the nitrile group remote from the chiral centre. Indeed, it is the case that most nitrilases only react in this way where there is the choice of reacting with a sterically hindered nitrile group e.g. in the vicinity of a chiral centre (but not necessarily at a chiral centre) and a unhindered nitrile group e.g. one remote from a chiral centre. Without wishing to be bound by theory, it is believed that in the case of a dinitrile which has a chiral centre at or close to the nitrile group, such as 2-methyl glutaronitrile, the non-chiral end i.e. the end which is more remote from any chiral carbon atom reacts more quickly than at the other end of the dinitrile which is closer to the chiral carbon atom. Without wishing to be bound by theory, this may be due to less steric hindrance.

Various prior art references describe nitrilases.

The Journal of Biotechnology 133 (2008), 327-333 describes the new nitrilase from Bradyrhizobium japonicum USDA 110 for gene cloning, biochemical characterisation and substrate specificity.

Applied Microbiology Biotechnology (1999) 52: 654-659 describes a gram-negative bacterium producing a heat-stable nitrilase highly active on aliphatic dinitriles.

The present invention seeks to provide a bio-catalytic process using nitrilases which is more efficient and cost-effective than conventional methods for producing dicarboxylic acids. It thus aims to use a biotransformation approach instead of a traditional inorganic chemistry approach. Another aim of the present invention is to provide a route to dicarboxylic acids from dinitriles which is more efficient than prior art methods. This process may have significant economic benefits. It has a further aim to provide a process which generates substantially less waste and/or uses few reagents and solvents than conventional methods. A further aim is to contribute to the sustainable green chemical industry by converting an existing waste stream into a valuable chemical product. It is another aim to utilise a process which avoids the need for harmful reagents and avoids the use of traditional organic solvents. A further aim is to reduce the energy burden of the transformation by conducting the process at a lower temperature than would be the case in a traditional inorganic transformation. Similarly, it is also an aim to reduce the energy burden by conducting the process at ambient pressure or near-ambient pressure. The present invention satisfies some or all of the above aims.

### SUMMARY ASPECTS

According to the present invention, there is provided a process for the production of a dicarboxylic acid comprising the step of:
reacting a composition comprising one or more aliphatic dinitriles wherein the or each aliphatic dinitrile contains from 2 to 20 carbon atoms or one or more aromatic dinitriles wherein the or each aromatic dinitrile contains from 6 to 20 carbon atoms with a nitrilase to produce a dicarboxylic acid, wherein the nitrilase comprises a CWE motif and, wherein the nitrilase when tested using the OPA Nitrilase Assay with 2-methylglutaronitrile as substrate has an % total hydrolysis of at least 45% or when tested using the OPA Nitrilase Assay with 4-cyanopentanoic acid (hereinafter "4-CPA") as substrate has an % total hydrolysis of at least 15%.

The process of the invention is thus a very convenient reaction. The reaction can be performed under relatively mild conditions.

In an embodiment, the nitrilase is a variant of SEQ ID No.1 and has an amino acid sequence which when aligned to SEQ ID No. 1 has a modification at one or more of the following positions: Val 90, Leu 131, and Thr243. The term "modification" as used herein refers to a substitution, deletion or addition at the recited amino acid position. Preferably, the modification is a substitution.

In another embodiment, the modification at Val 90 is V90A and/or the modification at Thr 243 is T243A. In an additional or alternative embodiment, the modification at Leu 131 is L131I, L131V, L131Y or L131M.

In a further embodiment, the nitrilase has at least 90% sequence identity to SEQ ID No. 1.

The nitrilase preferably has at least 90% sequence identity with a wild-type nitrilase from a *Bradyrhizobium* or *Nitratireductor* species.

In another embodiment, the nitrilase has an amino acid sequence which has at least 90% sequence identity to any one of: SEQ ID No. 2, SEQ ID No. 3 and SEQ ID No. 5.

The nitrilase is preferably immobilised. This may involve the use of a suitable support which could be a fixed bed or column, or may be in the form of plastic beads having suitable groups allowing for binding to the nitrilase. The beads may be magnetic in one embodiment for easy recovery from solution.

In another embodiment, an amidase is added prior, simultaneously or subsequent to reaction between the dinitrile and the nitrilase, preferably simultaneously. In a preferred embodiment the amidase is capable of reacting with 4-cyanopentanamide (hereinafter "4-CPAmide"). The amidase is preferably an aliphatic amidase (E.C. 3.5.1.4) which is a member of Pfam PF01425.

The conversion yield to a dicarboxylic acid is over 50%. Preferably it is at least 60%, and more preferably is at least 70% or at least 75%. Most preferably it is at least 80% or at least 90%.

In some embodiments, the process may be stopped when there is 75% conversion of the aliphatic dinitrile to dicarboxylic acid. In an alternative embodiment, the reaction is continued until there is at least 80%.

Usually the reaction is carried out at a pH in the range of 6 to 8.

In some embodiments, the dicarboxylic acid is chiral and the reaction yields an enantiomeric excess of 75% or more of one isomer of the dicarboxylic acid. Preferably the enantiomeric excess is 80% or more.

In some embodiments, the reaction yields an enantiomeric excess of one isomer of mononitrile. Preferably, the enantiomeric excess of is 70% or more.

The reaction may be carried out at a temperature between ambient room temperature i.e. from about 20°C or 25°C to 60°C. The reaction is preferably carried out at a temperature of 38-42°C.

The reaction is normally carried out over a period of 6 days or less, and more preferably less than 4 days.

In an embodiment, the source of the dinitrile contains more than one dinitrile compound. Alternatively, the source of the dinitrile contains only one dinitrile compound.

The source of the dinitrile may be a waste stream or the process may be applied to a pure or substantially pure (i.e. more than 90% or 95% pure) dinitrile source. Preferably the source of the dinitrile is a waste stream from an industrial process. In a preferred embodiment, the waste stream is a byproduct of the manufacture of adiponitrile (1,4-dicyanobutane).

In an embodiment, the or each dinitrile is an aliphatic dinitrile. More preferably, the or each aliphatic dinitrile contains from 5 to 10 carbon atoms, and preferably contains 5 or 6 carbon atoms.

The dinitrile may be 2-methylglutaronitrile and the dicarboxylic acid produced is 2-methylglutaric acid. Alternatively or in addition the dinitrile may be 2-ethylsuccinonitrile and the dicarboxylic acid produced is 2-ethylsuccinic acid.

In an alternative embodiment, the dinitrile is an aromatic dinitrile. Preferably, the or each aromatic dinitrile contains from 6 to 10 carbon atoms, and preferably contains 6, 7, or 8 carbon atoms.

The embodiments described above may be taken individually or they are applicable to the invention as a whole in any combination. Thus different combinations of some or all of the embodiments can be applied to the invention unless it is stated that particular embodiments are alternatives of one another.

The invention also provides a process for producing diesters comprising the steps of:
a. Producing a dicarboxylic acid in accordance with the present invention; and
b. Converting the dicarboxylic acid to a diester by reaction with one or more alcohols, wherein the or each alcohol contains from 1 to 20 carbon atoms.

The invention also provides the use of a nitrilase in the production carboxylic acids and/or dibasic esters, wherein the nitrilase comprises a CWE motif and, wherein the nitrilase when tested using the OPA Nitrilase Assay with 2-methylglutaronitrile as substrate has an % total hydrolysis of at least 45% or when tested using the OPA Nitrilase Assay with 4-cyanopentanoic acid as substrate has an % total hydrolysis of at least 15%. In an embodiment, the nitrilase is a variant of SEQ ID No.1 and has an amino acid sequence which when aligned to SEQ ID No. 1 has a modification at one or more of the following positions: Val 90, Leu 131 and Thr 243. In a further embodiment, the modification at Val 90 is V90A and/or the modification at Thr 243 is T243A. In an additional or alternative embodiment, the modification at Leu 131 is L131I, L131V, L131Y or L131M.

In an embodiment, the nitrilase has at least 90% sequence identity to SEQ ID No. 1.

In another embodiment, the nitrilase has at least 90% sequence identity with a wild-type nitrilase from a *Bradyrhizobium* or *Nitratireductor* species.

Alternatively, the nitrilase has an amino acid sequence which has at least 90% sequence identity to any one of: SEQ ID No. 2, SEQ ID No. 3 and SEQ ID No. 5.

The various embodiments described above in relation to the reaction are also applicable to the use described above. As before the embodiments may be taken individually or they are applicable to the invention as a whole in any combination. Thus different combinations of some or all of the embodiments can be applied to the invention unless it is stated that particular embodiments are alternatives of one another.

The invention also provides an isolated polypeptide having nitrilase activity, which comprises an amino acid sequence which when aligned to SEQ ID No. 1 has a modification at Val 90, Leu 131 and/or Thr243 and wherein:
a. the amino acid sequence has at least 90% sequence identity to SEQ ID NO. 1; or
b. the amino acid sequence binds under stringent conditions to the complement of SEQ ID No. 1.

The modification at Val 90 is V90A and/or the modification at Thr 243 is T243A. Additionally or alternatively, the modification at Leu 131 is L131I, L131V, L131Y or L131M.

A suitable nitrilase can be determined very easily by assay and the skilled person will immediately understand this. The nitrilase when tested using the OPA Nitrilase Assay with 2-methylglutaronitrile as substrate has a % total hydrolysis of at least 45%, or when tested using the OPA Nitrilase Assay with 4-cyanopentanoic acid as substrate has an % total hydrolysis of at least 15%.

The invention also provides a nucleic acid encoding an amino acid as described above.

### DETAILED DESCRIPTION

The term "nitrilase" as used herein means an enzyme having nitrilase activity (generally classified as E.C. 3.5.5.1 in accordance with the Enzyme Nomenclature Recommendations (1992) of the Nomenclature Committee of the International Union of Biochemistry and Molecular Biology). Suitably, a nitrilase for use in the present invention is capable of hydrolysing 2-methylglutaronitrile (2-MGN) to 2-methylglutaric acid (2-MGA) or is capable of hydrolysing both (S) and (R) enantiomers of 4-CPA to 2-MGA.

Pfam is a database of protein domain families. Pfam contains curated multiple sequence alignments for each family as well as profile hidden Markov models (profile HMMs) for identifying these domains in new sequences. An introduction to Pfam can be found in Bateman A et al. (2002) Nucleic Acids Res. 30; 276-280. Hidden Markov models are used in a number of databases that aim at classifying proteins, for review see Bateman A and Haft D H (2002) Brief Bioinform 3; 236-245.

For a detailed explanation of hidden Markov models and how they are applied in the Pfam database see Durbin R, Eddy S, and Krogh A (1998) Biological sequence analysis; probabilistic models of proteins and nucleic acids. Cambridge University Press, ISBN 0-521-62041-4. The Hammer software package can be obtained from Washington University, St Louis, USA.

Suitably, the nitrilase is a member of PF00795.

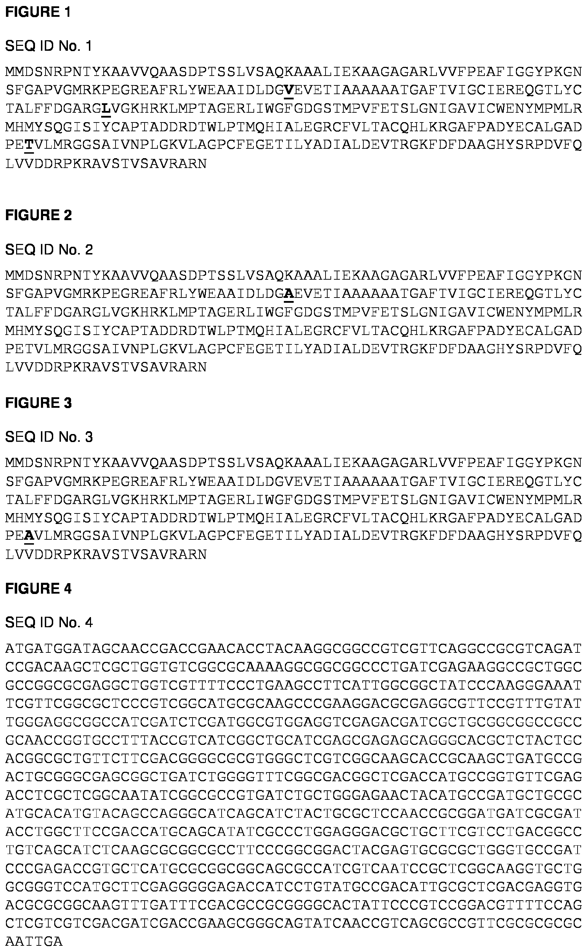

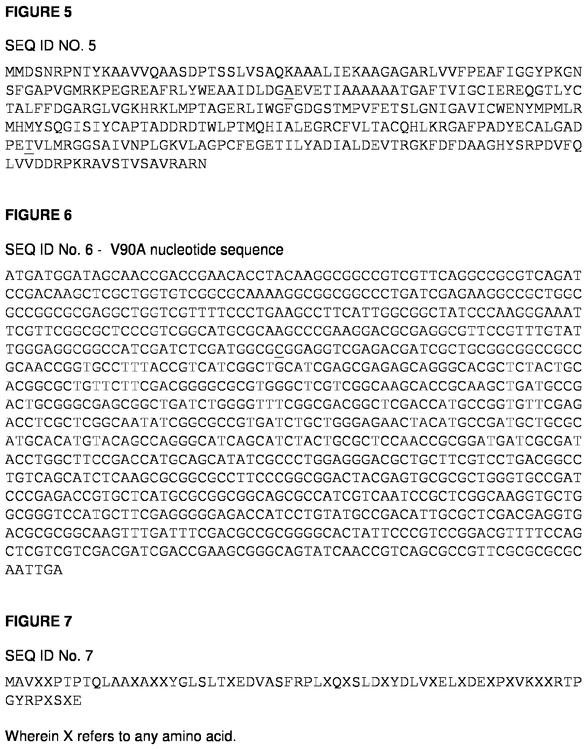

The nitrilase enzyme according to the present invention comprises the following catalytic site: Cys-171, Trp-172 and Glu-173 or comprises a cysteine residue, a tryptophan residue and glutamic acid residue, respectively, at positions corresponding to: Cys-171, Trp-172 and Glu-173 in the *Bradyrhizobium diazoefficiens* nitrilase enzyme shown in FIG. 1 (SEQ ID No. 1). Positions 90, 131 and 243 are underlined.

In one aspect, the nitrilase may be a variant nitrilase prepared by modifying a wild-type sequence.

Suitably, the wild-type nitrilase enzyme according to the present invention may be obtainable, preferably obtained, from organisms from one or more of the following genera *Bradyrhizobium* and *Nitratireductor.*

Suitably, the wild-type nitrilase enzyme according to the present invention may be obtainable, preferably obtained, *Bradyrhizobium* diazoefficiens (such as *Bradyrhizobium* diazoefficiens USDA 110) or *Nitratireductor aquibiodomus* (such as Nitratireductor aquibiodomus RA22).

Suitably, the wild-type enzyme has an amino acid sequence as shown in SEQ ID No.1 or has an amino acid sequence having at least 70, or at least 80% or at least 90% identity thereto.

Suitably, the nitrilase for use as disclosed herein when aligned with SEQ ID No. 1 does not have a modification at positions equivalent to any one or more of positions: 49, 53, 57, 61, 70, 120, 121, 138, 140, 141, 153, 173 and 245.

Suitably, the nitrilase may have the motif CWEN.

The position of a particular amino acid within a nitrilase according to the present invention is determined by alignment of the amino acid sequence of said nitrilase with SEQ ID No. 1 using a standard sequence alignment tool such as by alignment of two sequences using the Smith-Waterman algorithm, or with the CLUSTALW2 algorithms, wherein the sequences are said to be aligned when the alignment score is highest. Alignment scores may be calculated according to the methods described by Wilbur, W. J. and Lipman, D. J. (1983) Rapid similarity searches of nucleic acid and protein data banks . Proc. Natl. Acad. Sci. USA, 80: 726-730. Preferably default parameters are used in the ClustalW2 (1.82) algorithm: Protein Gap Open Penalty = 10.0; Protein Gap Extension Penalty = 0.2; Protein matrix = Gonnet; Protein/DNA ENDGAP = -1; Protein/DNA GAPDIST = 4.

Suitably, a position of a particular amino acid within a nitrilase according to the present invention may be determined by alignment of the amino acid sequence of the polypeptide with SEQ ID No. 1 using the AlignX program (part of the vectorNTI suite) with default parameters for multiple alignment (Gap opening penalty: 10 og Gap extension penalty 0.05).

Suitably, the variant nitrilase has an amino acid sequence which comprises or consists of an amino acid sequence having at least 90% (preferably at least 95%) identity to any one of SEQ ID No.s 2, 3 or 5. Suitably, the variant nitrilase may comprise or consist of an amino acid sequence as set forth in SEQ ID No.2, SEQ ID No. 3 or SEQ ID No. 5.

In the processes and uses of the present invention, an amidase may optionally be used in combination with a nitrilase. Suitably, the amidase may react with 4-CPAmide. Preferably, the amidase is an aliphatic amidase (E.C. 3.5.1.4) which is a member of Pfam PF01425. It has surprisingly been found that amidases which map closer to nitrilases in the genome have particular utility in the present invention. Suitably, an amidase for use in the present invention may comprise an amino sequence as set forth in SEQ ID No. 7.

### Protocol for the Determination of total % hydrolysis using the OPA assay with 2-MGN or 4-CPA as substrate

The OPA assay is a known and published method to determine hydrolysis of nitriles - see Black et al. 2015 ("A high-throughput screening method for determining the substrate scope of nitrilases", Chemical Communications, 51, 2015, pages 2660-2662).

For the OPA assay with 2-MGN, 2-MGN is used as a substrate at 5 mM. For the OPA assay with 4-CPA, 4-CPA is used as a substrate at 10 mM. Hence, in each assay both substrate solutions initially contain 10 mM hydrolysable nitrile groups, which would result in the detection of a maximum of 10 mM ammonia at complete hydrolysis.

Each value is a percentage of the maximum ammonia concentration. This is determined using a calibration graph obtained using a number of standard concentrations no less than four between 0 and 10mM of an ammonium chloride solution.
1.1 Preparation of OPA reagent. o-Phthalaldehyde (200 mg mL-1) is dissolved in MeOH before dilution (1:100) into sodium tetraborate buffer (15 mM, pH 9.5). The reagent is stored at 5 °C in the dark until use.
1.2 Microplate preparation. To a solution of either 5 mM 2-MGN or 10mM 4-CPA (in EtOH or DMSO, 23 µL) was added a CFE solution (0.56 mg mL-1 in potassium phosphate buffer [10 mM, pH 7.2], 207 µL, in which the nitrilase comprises 25% of the total protein). Plates are covered and incubated overnight (18 h, 37 °C).
1.3 Detection of ammonia. To a mixture of DMSO (140 µL) and OPA reagent (100 µL) is added the test solution (50 µL) and TCA solution (aq. 10% w/v, 50 µL). The resulting mixture is diluted into DMSO (1:2) and incubated (RT, 10 min). Absorbance of the solution at 675 nm is measured using a BioTek Synergy HT multi-mode microplate reader. Concentration of ammonia is determined by comparison to a standard curve prepared using standard aqueous solutions of NH4CI (2-12 mM) and deionised water as a blank.

The percentage total hydrolysis is calculated relative to the total ammonia that could be produced by 100% hydrolysis.

Alternatively, the % total hydrolysis can be measured using GC or HPLC.

In one embodiment, a nitrilase for use in the present invention is one which is capable of hydrolysing both nitriles of 2-MGN to produce 2-MGA. Suitably, the nitrilase may be capable of hydrolysing both enantiomers of 4-CPA such that both (R) and (S) methyl-4-cyanopenantoic acid is measurable following chemical esterification.

A person of ordinary skill in the art is readily able to measure the production of both (R) and (S) methyl-4-cyanopentoic acid.

4-CPA may be measured using HPLC. Alternatively, (R) and (S) methyl 4-cyanopentanoate and (R) and (S) methyl 2-methylglutarate (i.e. methyl esters of 4-CPA and 2-MGA which can be formed chemically on samples of 4-CPA and 2-MGA isolated from the reaction) may be measured using chiral GC

Nevertheless, illustrative parameters for such measurement is given below.

**Reverse Phase HPLC**

| | |
|---|---|
| Instrument: | Thermo Scientific Ultimate 3000 uHPLC |
| Column: | Agilent Zorbax SB-AQ (150mm x 4.6mm, 5µm particle size) |
| Mobile phase: | 0.1% aqueous acetic acid + acetonitrile (88:12) |
| Flow rate: | 1ml/min |
| Colum temp: | 30oC |
| Sample injection volume: | 10µl |
| Detection: | UV at 210nm |

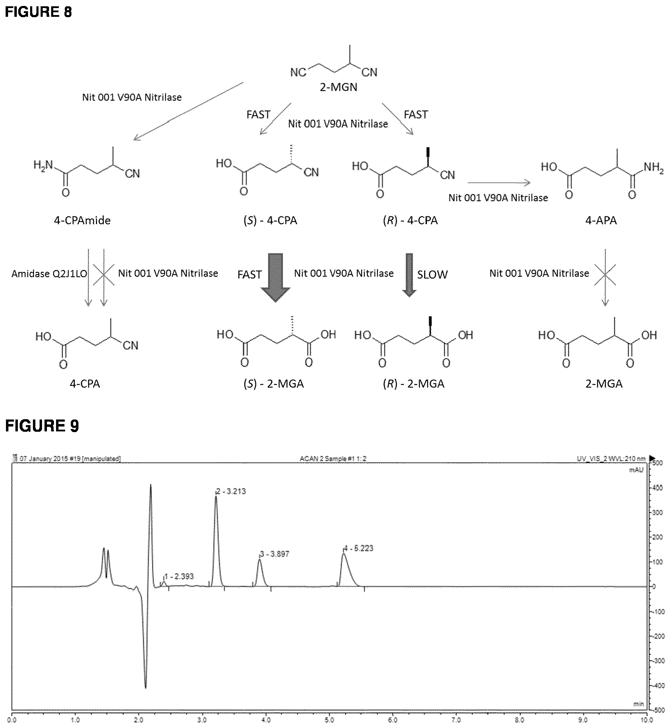

Retention Times: 4-Amidopentanoic acid (2.4 minutes), 4-Cyanopentanamide (3.2 minutes), 2-Methyl glutaric acid (3.9 minutes), 4-Cyanopentanoic acid (5.2 minutes). See Figure 9.

**Chiral GC**

| | |
|---|---|
| Column: | CP-ChiraSil-DEX CB 25m x 0.32mm x 0.25µm film thickness |
| Temperature: | 65oC to 120oC at 1.5oC/min and hold 2minutes |
| Run Time: | 38 minutes |
| Inlet Temp: | 200oC |
| Carrier gas: | Helium @2ml/min |
| Split ratio: | 100:1 |
| Injection volume: | 1µl |
| Detector: gas | FID 300oC, 30mL/min H2, 400mL/min Air, 25mL/min He makeup |

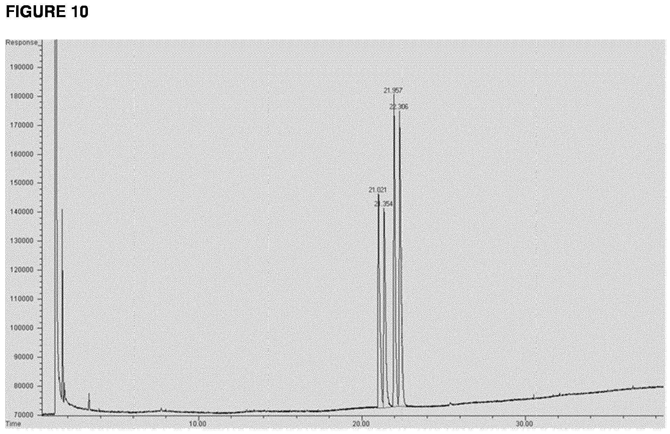

Retention Times: (R) - Methyl 4-cyanopentanoate (21.0 minutes), (S) - Methyl 4-cyanopentanoate (21.0 minutes), (S) - Methyl 2-methylglutarate (21.9 minutes), (R) - Methyl 2-methylglutarate (22.3 minutes). See Figure 10.

Suitably, the variant nitrilase has increased hydrolysis activity on 2-MGN and/or 4-CPA as compared with the wild type enzyme (SEQ ID No. 1). In this context, an increase in hydrolysis may be measured by a reduction of (R) 4-CPA and/or (S) 4-CPA compared to the wild type under identical reaction conditions. In addition or in the alternative, increased hydrolysis may be measured by increased production of (S) 2-MGA and/or (R) 2-MGA compared to the wild type enzyme under identical conditions.

In one embodiment, when utilising chiral GC as disclosed above and following isolation of 4-CPA and 2-MGA from the nitrilase reaction and their subsequent chemical esterification to form the methyl esters of 4-CPA and 2-MGA, an increase in hydrolysis may be measured by a reduction of methyl 4-cyanopentanoate compared to the wild-type enzymes under identical conditions. In addition or in the alternative, increased hydrolysis may be measured by increased production methyl 2-methylglutarate compared to the wild-type enzyme under identical conditions.

In one embodiment, the enzyme according to the present invention is preferably not immobilised, in particular is not immobilised on a solid support.

In an alternative embodiment, the enzyme may be immobilised.

Immobilised nitrilases and/or amidases can be prepared using immobilisation techniques known in the art. There are numerous methods of preparing immobilised enzymes, which will be apparent to a person skilled in the art (for example the techniques referred to in EP 0 746 608; or Balcao V M, Paiva A L, Malcata F X., Enzyme Microb Technol. 1996 May 1; 18(6):392-416; or Reetz M T, Jaeger K E. Chem Phys Lipids. 1998 June; 93(1-2):3-14; or Bornscheuer U T, Bessler C, Srinivas R, Krishna S H. Trends Biotechnol. 2002 October; 20(10):433-7 (each of which is incorporated herein by reference). This may involve the use of a suitable support which could be a fixed bed or column, or may be in the form of plastic beads having suitable groups allowing for binding to the nitrilase. The beads may be magnetic in one embodiment for easy recovery from solution. Suitably, an enzyme for use in the present invention may be immobilised as CLEAs (cross-linked enzyme aggregates). Methods for the productions of CLEAs are known in the art - see for example Sheldon ("Cross-linked enzyme aggregates (CLEAs): stable and recyclable biocatalysts, 7th International Conference on Protein Stabilization 2007, pages 1583-1587, doi: 10. 1042/BST0351583). Suitably, when an amidase is used in the methods disclosed herein, both the nitrilase and amidase may be in the form of a combi-CLEA.

In one aspect, suitably the nitrilase and/or amidase for use in the present invention may be in an isolated form. The term "isolated" means that the sequence is at least substantially free from at least one other component with which the sequence is naturally associated in nature and as found in nature.

In one aspect, suitably the nitrilase and/or amidase for use in the present invention may be in a purified form. The term "purified" means that the sequence is in a relatively pure state-e.g. at least about 51% pure, or at least about 75%, or at least about 80%, or at least about 90% pure, or at least about 95% pure or at least about 98% pure.

The present invention provides nucleic acids encoding nitrilases or amidases as disclosed herein. A nucleotide sequence encoding either a polypeptide which has the specific properties as defined herein or a polypeptide which is suitable for modification may be isolated from any cell or organism producing said polypeptide. Various methods are well known within the art for the isolation of nucleotide sequences.

For example, a genomic DNA and/or cDNA library may be constructed using chromosomal DNA or messenger RNA from the organism producing the polypeptide. If the amino acid sequence of the polypeptide is known, labelled oligonucleotide probes may be synthesised and used to identify polypeptide-encoding clones from the genomic library prepared from the organism. Alternatively, a labelled oligonucleotide probe containing sequences homologous to another known polypeptide gene could be used to identify polypeptide-encoding clones. In the latter case, hybridisation and washing conditions of lower stringency are used.

Alternatively, polypeptide-encoding clones could be identified by inserting fragments of genomic DNA into an expression vector, such as a plasmid, transforming enzyme-negative bacteria with the resulting genomic DNA library, and then plating the transformed bacteria onto agar containing an enzyme inhibited by the polypeptide, thereby allowing clones expressing the polypeptide to be identified.

In a yet further alternative, the nucleotide sequence encoding the polypeptide may be prepared synthetically by established standard methods, e.g. the phosphoroamidite method described by Beucage S. L. et al (1981) Tetrahedron Letters 22, p 1859-1869, or the method described by Matthes et al (1984) EMBO J. 3, p 801-805. In the phosphoroamidite method, oligonucleotides are synthesised, e.g. in an automatic DNA synthesiser, purified, annealed, ligated and cloned in appropriate vectors.

The nucleotide sequence may be of mixed genomic and synthetic origin, mixed synthetic and cDNA origin, or mixed genomic and cDNA origin, prepared by ligating fragments of synthetic, genomic or cDNA origin (as appropriate) in accordance with standard techniques. Each ligated fragment corresponds to various parts of the entire nucleotide sequence. The DNA sequence may also be prepared by polymerase chain reaction (PCR) using specific primers, for instance as described in U.S. Pat. No. 4,683,202 or in Saiki R K et al (Science (1988) 239, pp 487-491).

The term "nucleotide sequence" as used herein refers to an oligonucleotide sequence or polynucleotide sequence, and variant, homologues, fragments and derivatives thereof (such as portions thereof). The nucleotide sequence may be of genomic or synthetic or recombinant origin, which may be double-stranded or single-stranded whether representing the sense or antisense strand. The term "nucleotide sequence" in relation to the present invention includes genomic DNA, cDNA, synthetic DNA, and RNA. Preferably it means DNA, more preferably cDNA for the coding sequence.

In a preferred embodiment, the nucleotide sequence *per se* encoding a nitrilase of the invention or for use in the present invention is a non-native sequence. A non-native sequence does not cover the native nucleotide sequence in its natural environment when it is linked to its naturally associated sequence(s) that is/are also in its/their natural environment. The polypeptide of the present invention can be expressed by a nucleotide sequence in its native organism but wherein the nucleotide sequence is not under the control of the promoter with which it is naturally associated within that organism.

Preferably the polypeptide is not a native polypeptide. In this regard, the term "native polypeptide" means an entire polypeptide that is in its native environment and when it has been expressed by its native nucleotide sequence.

Typically, the nucleotide sequence encoding polypeptides having the specific properties as defined herein is prepared using recombinant DNA techniques (i.e. recombinant DNA). However, in an alternative embodiment of the invention, the nucleotide sequence could be synthesised, in whole or in part, using chemical methods well known in the art (see Caruthers M H et al (1980) Nuc Acids Res Symp Ser 215-23 and Horn T et al (1980) Nuc Acids Res Symp Ser 225-232).

Once an enzyme-encoding nucleotide sequence has been isolated, or a putative enzyme-encoding nucleotide sequence has been identified, it may be desirable to modify the selected nucleotide sequence, for example it may be desirable to mutate the sequence in order to prepare an enzyme in accordance with the present invention.

Mutations may be introduced using synthetic oligonucleotides. These oligonucleotides contain nucleotide sequences flanking the desired mutation sites.

A suitable method is disclosed in Morinaga et al (Biotechnology (1984) 2, p 646-649). Another method of introducing mutations into enzyme-encoding nucleotide sequences is described in Nelson and Long (Analytical Biochemistry (1989), 180, p 147-151).

Instead of site directed mutagenesis, such as described above, one can introduce mutations randomly for instance using a commercial kit such as the GeneMorph PCR mutagenesis kit from Stratagene, or the Diversify PCR random mutagenesis kit from Clontech. EP 0 583 265 refers to methods of optimising PCR based mutagenesis, which can also be combined with the use of mutagenic DNA analogues such as those described in EP 0 866 796.

Thus, it is possible to produce numerous site directed or random mutations into a nucleotide sequence, either in vivo or in vitro, and to subsequently screen for improved functionality of the encoded polypeptide by various means. Using in silico and exo mediated recombination methods (see WO 00/58517, U.S. Pat. No. 6,344,328, U.S. Pat. No. 6,361,974), for example, molecular evolution can be performed where the variant produced retains very low homology to known enzymes or proteins. Such variants thereby obtained may have significant structural analogy to known transferase enzymes, but have very low amino acid sequence homology.

As a non-limiting example, in addition, mutations or natural variants of a polynucleotide sequence can be recombined with either the wild type or other mutations or natural variants to produce new variants. Such new variants can also be screened for improved functionality of the encoded polypeptide. As will be apparent to a person skilled in the art, using molecular evolution tools an enzyme may be altered to improve the functionality of the enzyme.

Suitably, the nitrilase used in the invention may be a variant, i.e. may contain at least one amino acid substitution, deletion or addition, when compared to a parental enzyme. Variant enzymes of the present invention retain at least 60%, 70%, 80%, 90%, 95%, 97%, or 99% sequence identity with the parent enzyme. In one aspect, suitable parent enzymes may include any nitrilase enzyme capable of hydrolysing 2-MGN to 2-MGA. Preferably, the parent enzyme is a member of Pfam PF00795.

Suitably, the nitrilase may be a variant with enhanced enzyme activity on the chiral end of 2-MGN and/or 4-CPA. Suitably, the nitrilase may be a variant with enhanced enzyme activity on the (R) enantiomer of 2-MGN and/or 4-CPA.

As used herein, the term "amino acid sequence" is synonymous with the term "polypeptide" and/or the term "protein". In some instances, the term "amino acid sequence" is synonymous with the term "peptide". The amino acid sequence may be prepared/isolated from a suitable source, or it may be made synthetically or it may be prepared by use of recombinant DNA techniques. Suitably, the amino acid sequences may be obtained from the isolated polypeptides taught herein by standard techniques.

The present invention also encompasses the use of sequences having a degree of sequence identity with amino acid sequence(s) of a polypeptide having the specific properties defined herein or of any nucleotide sequence encoding such a polypeptide (hereinafter referred to as a "homologous sequence(s)"). Here, the term "homologue" means an entity having a certain homology with the subject amino acid sequences and the subject nucleotide sequences. Here, the term "homology" can be equated with "identity".

The homologous amino acid sequence and/or nucleotide sequence should provide and/or encode a polypeptide which retains the functional activity and/or enhances the activity of the enzyme.

In the present context, a homologous sequence is taken to include an amino acid sequence which may be at least 75, 85 or 90% identical, preferably at least 95 or 98% identical to the subject sequence. Typically, the homologues will comprise the same active sites etc. as the subject amino acid sequence. Although homology can also be considered in terms of similarity (i.e. amino acid residues having similar chemical properties/functions), in the context of the present invention it is preferred to express homology in terms of sequence identity.

In the present context, a homologous sequence is taken to include a nucleotide sequence which may be at least 75, 85 or 90% identical, preferably at least 95 or 98% identical to a nucleotide sequence encoding a polypeptide of the present invention (the subject sequence). Typically, the homologues will comprise the same sequences that code for the active sites etc. as the subject sequence. Although homology can also be considered in terms of similarity (i.e. amino acid residues having similar chemical properties/functions), in the context of the present invention it is preferred to express homology in terms of sequence identity.

Suitably, percentage identity may be calculated using the multiple alignment feature in DNASIS™ (Hitachi Software), based on an algorithm, analogous to CLUSTAL (Higgins D G & Sharp P M (1988), Gene 73(1), 237-244). Once the software has produced an optimal alignment, it is possible to calculate % sequence identity.

The sequences may also have deletions, insertions or substitutions of amino acid residues which produce a silent change and result in a functionally equivalent substance. Deliberate amino acid substitutions may be made on the basis of similarity in polarity, charge, solubility, hydrophobicity, hydrophilicity, and/or the amphipathic nature of the residues as long as the secondary binding activity of the substance is retained. For example, negatively charged amino acids include aspartic acid and glutamic acid; positively charged amino acids include lysine and arginine; and amino acids with uncharged polar head groups having similar hydrophilicity values include leucine, isoleucine, valine, glycine, alanine, asparagine, glutamine, serine, threonine, phenylalanine, and tyrosine.

The present invention also encompasses homologous substitution (substitution and replacement are both used herein to mean the interchange of an existing amino acid residue, with an alternative residue) that may occur i.e. like-for-like substitution such as basic for basic, acidic for acidic, polar for polar etc. Non-homologous substitution may also occur i.e. from one class of residue to another or alternatively involving the inclusion of unnatural amino acids such as ornithine (hereinafter referred to as Z), diaminobutyric acid ornithine (hereinafter referred to as B), norleucine ornithine (hereinafter referred to as O), pyriylalanine, thienylalanine, naphthylalanine and phenylglycine.

Replacements may also be made by unnatural amino acids.

Nucleotide sequences for use in the present invention or encoding a polypeptide having the specific properties defined herein may include within them synthetic or modified nucleotides. A number of different types of modification to oligonucleotides are known in the art. These include methylphosphonate and phosphorothioate backbones and/or the addition of acridine or polylysine chains at the 3' and/or 5' ends of the molecule. For the purposes of the present invention, it is to be understood that the nucleotide sequences described herein may be modified by any method available in the art. Such modifications may be carried out in order to enhance the in vivo activity or life span of nucleotide sequences.

The present invention also encompasses the use of nucleotide sequences that are complementary to the sequences discussed herein, or any derivative, fragment or derivative thereof. If the sequence is complementary to a fragment thereof then that sequence can be used as a probe to identify similar coding sequences in other organisms etc.

Polynucleotides such as DNA polynucleotides and probes according to the invention may be produced recombinantly, synthetically, or by any means available to those of skill in the art. They may also be cloned by standard techniques.

The present invention also encompasses sequences that are complementary to the sequences of the present invention or sequences that are capable of hybridising either to the sequences of the present invention or to sequences that are complementary thereto.

The term "hybridisation" as used herein shall include "the process by which a strand of nucleic acid joins with a complementary strand through base pairing" as well as the process of amplification as carried out in polymerase chain reaction (PCR) technologies.

The present invention also encompasses the use of nucleotide sequences that are capable of hybridising to the sequences that are complementary to the subject sequences discussed herein, or any derivative, fragment or derivative thereof. The present invention also encompasses sequences that are complementary to sequences that are capable of hybridising to the nucleotide sequences discussed herein.

Hybridisation conditions are based on the melting temperature (Tm) of the nucleotide binding complex, as taught in Berger and Kimmel (1987, Guide to Molecular Cloning Techniques, Methods in Enzymology, Vol. 152, Academic Press, San Diego Calif.), and confer a defined "stringency" as explained below.

Maximum stringency typically occurs at about Tm-5° C. (5° C. below the Tm of the probe); high stringency at about 5° C. to 10° C. below Tm; intermediate stringency at about 10° C. to 20° C. below Tm; and low stringency at about 20° C. to 25° C. below Tm. As will be understood by those of skill in the art, a maximum stringency hybridisation can be used to identify or detect identical nucleotide sequences while an intermediate (or low) stringency hybridisation can be used to identify or detect similar or related polynucleotide sequences.

Preferably, the present invention encompasses sequences that are complementary to sequences that are capable of hybridising under high stringency conditions or intermediate stringency conditions to nucleotide sequences encoding polypeptides having the specific properties as defined herein.

More preferably, the present invention encompasses sequences that are complementary to sequences that are capable of hybridising under high stringent conditions (e.g. 65° C. and 0.1 ×SSC {1×SSC=0.15 M NaCl, 0.015 M Na-citrate pH 7.0}) to nucleotide sequences encoding polypeptides having the specific properties as defined herein.

The present invention also relates to nucleotide sequences that can hybridise to the nucleotide sequences discussed herein (including complementary sequences of those discussed herein).

The present invention also relates to nucleotide sequences that are complementary to sequences that can hybridise to the nucleotide sequences discussed herein (including complementary sequences of those discussed herein).

In a preferred aspect, the present invention covers nucleotide sequences that can hybridise to the nucleotide sequences discussed herein, or the complement thereof, under stringent conditions (e.g. 50° C. and 0.2xSSC) or under high stringent conditions (e.g. 65° C. and 0.1 xSSC).

A nucleotide sequence for use in the present invention or for encoding a polypeptide having the specific properties as defined herein can be incorporated into a recombinant replicable vector. The vector may be used to replicate and express the nucleotide sequence, in polypeptide form, in and/or from a compatible host cell. Expression may be controlled using control sequences which include promoters/enhancers and other expression regulation signals. Prokaryotic promoters and promoters functional in eukaryotic cells may be used. Tissue specific or stimuli specific promoters may be used. Chimeric promoters may also be used comprising sequence elements from two or more different promoters described above.

The polypeptide produced by a host recombinant cell by expression of the nucleotide sequence may be secreted or may be contained intracellularly depending on the sequence and/or the vector used. The coding sequences can be designed with signal sequences which direct secretion of the substance coding sequences through a particular prokaryotic or eukaryotic cell membrane.

The term "expression vector" means a construct capable of in vivo or in vitro expression.

Preferably, the expression vector is incorporated in the genome of the organism. The term "incorporated" preferably covers stable incorporation into the genome.

The nucleotide sequence of the present invention or coding for a polypeptide having the specific properties as defined herein may be present in a vector, in which the nucleotide sequence is operably linked to regulatory sequences such that the regulatory sequences are capable of providing the expression of the nucleotide sequence by a suitable host organism, i.e. the vector is an expression vector. Such vectors may be transformed into a suitable host cell to provide for expression of a polypeptide having the specific properties as defined herein.

The vectors may contain one or more selectable marker genes-such as a gene which confers antibiotic resistance e.g. ampicillin, kanamycin, chloramphenicol or tetracyclin resistance.

Thus, in a further embodiment, the invention provides a method of making nucleotide sequences of the present invention or nucleotide sequences encoding polypeptides having the specific properties as defined herein by introducing a nucleotide sequence into a replicable vector, introducing the vector into a compatible host cell, and growing the host cell under conditions which bring about replication of the vector.

In some applications, a nucleotide sequence for use in the present invention or a nucleotide sequence encoding a polypeptide having the specific properties as defined herein may be operably linked to a regulatory sequence which is capable of providing for the expression of the nucleotide sequence, such as by the chosen host cell. By way of example, the present invention covers a vector comprising the nucleotide sequence of the present invention operably linked to such a regulatory sequence, i.e. the vector is an expression vector.

The term "operably linked" refers to a juxtaposition wherein the components described are in a relationship permitting them to function in their intended manner. A regulatory sequence "operably linked" to a coding sequence is ligated in such a way that expression of the coding sequence is achieved under conditions compatible with the control sequences.

The term "regulatory sequences" includes promoters and enhancers and other expression regulation signals.

Suitably, the polypeptides of the invention or for use in the invention (such as a nitrilase and/or amidase) may be linked to another peptide. This peptide may involve a marker which, for example, may facilitate purification of the desired polypeptide e.g. in subsequent chromatography steps. A preferred marker for this purpose is a his-tag constructed from 6 monomer histidine units.

The term "promoter" is used in the normal sense of the art, e.g. an RNA polymerase binding site.

Enhanced expression of the nucleotide sequence encoding the enzyme having the specific properties as defined herein may also be achieved by the selection of heterologous regulatory regions, e.g. promoter, secretion leader and terminator regions.

Preferably, the nucleotide sequence of the present invention may be operably linked to at least a promoter.

Examples of suitable promoters for directing the transcription of the nucleotide sequence in a bacterial, fungal or yeast host are well known in the art.

The term "construct"-which is synonymous with terms such as "conjugate", "cassette" and "hybrid"-includes a nucleotide sequence encoding a polypeptide having the specific properties as defined herein for use according to the present invention directly or indirectly attached to a promoter. An example of an indirect attachment is the provision of a suitable spacer group such as an intron sequence, such as the Sh1-intron or the ADH intron, intermediate the promoter and the nucleotide sequence of the present invention. The same is true for the term "fused" in relation to the present invention which includes direct or indirect attachment. In some cases, the terms do not cover the natural combination of the nucleotide sequence coding for the protein ordinarily associated with the wild type gene promoter and when they are both in their natural environment.

The construct may even contain or express a marker which allows for the selection of the genetic construct.

For some applications, preferably the construct comprises at least a nucleotide sequence of the present invention or a nucleotide sequence encoding a polypeptide having the specific properties as defined herein operably linked to a promoter.

The term "host cell"-in relation to the present invention includes any cell that comprises either a nucleotide sequence encoding a polypeptide having the specific properties as defined herein or an expression vector as described above and which is used in the recombinant production of a polypeptide having the specific properties as defined herein.

Thus, a further embodiment of the present invention provides host cells transformed or transfected with a nucleotide sequence of the present invention or a nucleotide sequence that expresses a polypeptide having the specific properties as defined herein. The cells will be chosen to be compatible with the said vector and may for example be prokaryotic (for example bacterial), fungal, yeast or plant cells. Preferably, the host cells are not human cells.

Examples of suitable bacterial host organisms are gram negative bacterium or gram positive bacteria. Examples of suitable prokaryotic hosts include *E*. *coli* and *Bacillus subtilis.*

In one aspect, the present invention provides a process for the production of a dicarboxylic acid comprising the step of:
reacting a composition comprising one or more aliphatic dinitriles wherein the or each aliphatic dinitrile contains from 2 to 20 carbon atoms or one or more aromatic dinitriles wherein the or each aromatic dinitrile contains from 6 to 20 carbon atoms with a nitrilase to produce a dicarboxylic acid, wherein the nitrilase comprises a CWE motif and, wherein the nitrilase when tested using the OPA Nitrilase Assay with 2-methylglutaronitrile as substrate has an % total hydrolysis of at least 45% or when tested using the OPA Nitrilase Assay with 4-CPA as substrate has an % total hydrolysis of at least 15%.

Suitably, the process may be an industrial process and may be a large scale process. Suitably, the process may be capable of being conducted at higher than 500L capacity.

Suitably, the reaction step may be carried out at any temperature at which the nitrilase (and, optionally amidase) is active. Preferably, the reaction is carried out at a temperature in the range of about 30°C to about 50°C, preferably at a temperature in the range of about 35°C to about 45°C. In one aspect, the reaction step is carried out at about 42°C.

Suitably, the reaction may be carried out at a pH in the range of 6 to 8, preferably at a pH of about 7.

Suitably, the reaction mixture may comprise one or more buffering agents.

Suitably, the reaction step is continued for sufficient time to provide the desired yield of dicarboxylic acid. Advantageously, the nitrilases of the present invention can hydrolyse both nitriles groups and may yield conversion rates higher than 50%, preferably higher than 60%, preferably higher than 75%. Suitably, the duration of the reacting step may be up to 7 days. In one aspect, the reaction step is carried out for about 4 to about 6 days, preferably about 6 days.

Figure 8 shows the reaction scheme for the production of a specific dicarboxylic acid (2-MGA) from a specific aliphatic dinitrile (2-MGN). However, a skilled person would readily understand that this scheme may easily be adapted for any aliphatic dinitrile having 2 to 20 carbon atoms.

As can be seen, a nitrilase of the present invention may be capable of hydrolysing both the non-chiral end and both enantiomers of the chiral end of an aliphatic dintrile, such that the enzyme catalyses the production of a dicarboxylic acid.

Advantageously, this may yield a dicarboxylic acid rich in one enantiomer. For example, a variant nitrilase of the invention may react on (S) 4-CPA quicker than on (R) 4-CPA, hence the method of the invention could be used to generate a reaction product enriched for (S) MGA when 2-MGN is used as a substrate.

In one aspect, the reacting step is continued until there is up to 20% conversion to dicarboxylic acid, such as 10-20% dicarboxylic acid. For example, at low overall conversions (<20%) to 2-MGA, 2-MGA may advantageously be in a ratio of 90:10 % (S) to (R) enantiomers. Hence, 2-MGA may be enriched in the (S) enantiomer.

In another aspect, the reaction step is stopped when there is an enantiomeric excess of one isomer of mononitrile (or monocarboxylic acid). As illustrated in Figure 8, as the reaction progresses to higher conversions the (S) enantiomer of 4-CPA is hydrolysed significantly faster than the (R) enantiomer such that it is possible to obtain 4-CPA highly enriched in the (R) enantiomer (observed 99:1 ratio of (R) to (S)) at 55% conversion.

Hence, suitably, the process of the present invention may be one which yields an enantiomeric excess of at least 70%, or at least 80%, or at least 90%, or at least 95% of one isomer of a mononitrile (e.g. (R) 4-CPA).

### EXAMPLES

### EXAMPLE 1

A nitrilase from Bradyrhizobium japonicum USDA 110 having an amino acid sequence as set forth in SEQ ID No.1 was utilised. Following rounds of random mutagenesis of the nitrilase (SEQ ID No. 1) samples were sequenced and alanine scanning mutant data was conducted at positions of interest, said mutants generated to have one substitution to alanine at defined positions. The mutant nitrilases were tested to determine % nitrilase activity using the assays and methodology as detailed in Black et al. ("A high-throughput screening method for determining the substrate scope of nitrilases" Chem. Commun., 2015,51, 2660-2662, incorporated herein by reference) with 5 mM 2-MGN used as the substrate.

The results are presented in Table 1 below.

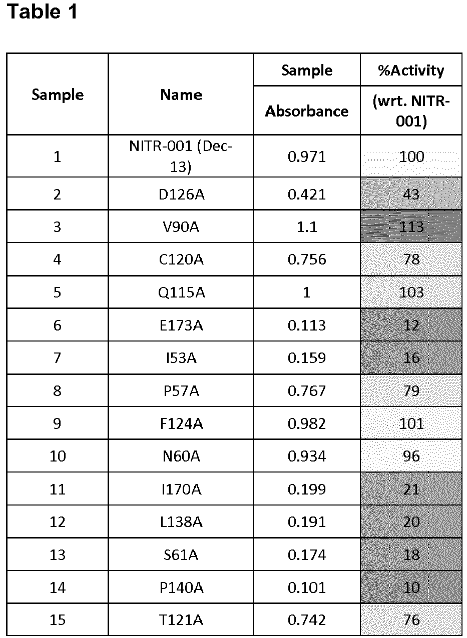

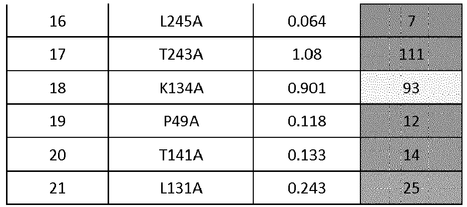

As can be seen, modification of positions 90 and/or 243 resulted in enhanced activity compared to the naturally occurring parent enzyme.

### EXAMPLE 2

Culture free extracts comprising a variant nitrilase as set forth in SEQ ID No. 2 (i.e. differing from the nitrilase of SEQ ID No.1 by the substitution of valine to alanine at position 90) were prepared and analysed in accordance with the methods disclosed in Black et al. ("A high-throughput screening method for determining the substrate scope of nitrilases" Chem. Commun., 2015,51, 2660-2662) incorporated herein by reference. 2-MGN was utilised as the substrate.

The results are presented in Table 2 below. As can be seen, the variant V90A hydrolysed both nitrile groups of 2-MGN under the defined conditions.

**Table 2**

| | **Absorbance** | | | |
|---|---|---|---|---|
| **CFE** | **EtOH Avg.** | **2-MGN Avg.** | **Corrected** | **%Conversion** |
| UNN Sept 2013 | 0.164 | 1.054 | 0.890 | 63 |
| UNN Dec 2013 | 0.296 | 1.107 | 0.812 | 58 |

### EXAMPLE 3

Tables 3 and 4 respectively contains the results of a standard OPA assay (as detailed in Black et al. ("A high-throughput screening method for determining the substrate scope of nitrilases" Chem. Commun., 2015,51, 2660-2662) incorporated herein by reference) using 2-MGN as a substrate at 5 mM and 4-CPA as a substrate at 10 mM (in triplicate as single substrate reactions, therefore six replicates for each CFE). Both substrate solutions initially contained 10 mM hydrolysable nitrile groups, which would result in the detection of a maximum of 10 mM ammonia at complete hydrolysis.

Each value is a percentage of the maximum ammonia concentration. This is determined from the ratio of the average sample absorbance to the average absorbance of a 10 mM ammonium chloride standard solution.

Table 3 has each CFE ordered for percent total nitrile groups hydrolysed using 2-MGN as a substrate, and Table 4 has each CFE ordered for percent total nitrile groups hydrolysed using 4-CPA as a substrate.

Codes for each may be interpreted as follows: NU Northumbria University, BC Biocatalysts, Nit# Nitrilase number #, WT Wild Type, OPT Codon-optimised,V90A Valine 90 substituted by Alanine (other single letter codes apply).

Final digits are a batch number.

**Table 3**

| Utilising the OPA nitrilase assay with 2-MGN as substrate yielded the following results | | |
|---|---|---|
| CFE No. | Code | %Total hydrolysis for 2-MGN |
| 9 | BC-Nit1-V90A-9645514 | 53 |
| 20 | NU-Nit1-L138M-0914 | 48 |
| 8 | NU-Nit1-V90A-1213 | 47 |
| 26 | NU-Nit1-L131I-0914 | 46 |
| 5 | BC-Nit1-WT-10647914 | 43 |
| 25 | NU-Nit1-L131V-0914 | 43 |
| 24 | NU-Nit1-L131Y-1014 | 42 |
| 23 | NU-Nit1-L131M-0914 | 41 |
| 4 | NU-Nit1-WT-0914 | 41 |

As can be seen, modification of position 90 significantly increased the total hydrolysis of 2-MGN.

**Table 4**

| Utilising the OPA nitrilase assay with the 2-MGN hydrolysis intermediate 4-CPA yielded the following results. | | |
|---|---|---|
| CFE No. | Code | %Total hydrolysis for 4-CPA |
| 9 | BC-Nit1-V90A-9645514 | 39 |
| 26 | NU-Nit1-L131I-0914 | 25 |
| 8 | NU-Nit1-V90A-1213 | 23 |
| 20 | NU-Nit1-L138M-0914 | 20 |
| 25 | NU-Nit1-L131V-0914 | 18 |
| 23 | NU-Nit1-L131M-0914 | 16 |
| 22 | NU-Nit1-L138V-1014 | 14 |
| 24 | NU-Nit1-L131Y-1014 | 13 |
| 14 | NU-Nit1-R129A-0714 | 11 |
| 5 | BC-Nit1-WT-10647914 | 9 |
| 3 | NU-Nit1-WT-1213 | 5 |
| 18 | NU-Nit1-T102A-0714 | 5 |
| 21 | NU-Nit1-L138I-1014 | 5 |
| 4 | NU-Nit1-WT-0914 | 5 |
| 2 | NU-Nit1-WT-0913 | 5 |

As can be seen, modification of position 90 significantly increased the total hydrolysis of 4-CPA.

The present application and invention further includes the subject matter of the following numbered clauses.
1. A process for the production of a dicarboxylic acid comprising the step of:
   i) reacting a composition comprising one or more aliphatic dinitriles wherein the or each aliphatic dinitrile contains from 2 to 20 carbon atoms or one or more aromatic dinitriles wherein the or each aromatic dinitrile contains from 6 to 20 carbon atoms with a nitrilase to produce a dicarboxylic acid, wherein the nitrilase comprises a CWE motif and, wherein the nitrilase when tested using the OPA Nitrilase Assay with 2-methylglutaronitrile as substrate has an % total hydrolysis of at least 45% or when tested using the OPA Nitrilase Assay with 4-CPA as substrate has an % total hydrolysis of at least 15%.
2. The process according to clause 1 wherein the nitrilase is a variant of SEQ ID No.1 and has an amino acid sequence which when aligned to SEQ ID No. 1 has a modification at Val 90, Leu 131 and/or Thr243.
3. The process according to clause 1 or clause 2, wherein the modification at Val 90 is V90A; the modification at Leu 131 is L131I, L131V, L131Y or L131M; and/or the modification at Thr 243 is T243A.
4. The process according to any one of clauses 1 to 3, wherein the nitrilase has at least 90% sequence identity to SEQ ID No. 1
5. The process according to any preceding clause wherein the reaction is carried out at a temperature of 40-45°C.
6. The process according to any preceding clause wherein the nitrilase has at least 90% sequence identity with a wild-type nitrilase from a Bradyrhizobium or Nitratireductor species.
7. The process according to any preceding clause, wherein the nitrilase has an amino acid sequence which has at least 90% sequence identity to any one of: SEQ ID No. 2, SEQ ID No. 3 and SEQ ID No. 5.
8. The process according to any preceding clause, wherein the nitrilase is immobilised.
9. The process according to any one of the preceding clauses, wherein an amidase is added prior, simultaneously or subsequent to reaction step i).
10. The process according to clause 8, wherein the amidase is capable of reacting with 4-CPAmide.
11. The process according to clause 8 or clause 9 wherein the amidase is an aliphatic amidase (E.C. 3.5.1.4) which is a member of Pfam PF01425.
12. The process according to any one of clauses 9 to 11, wherein the amidase comprises a sequence set forth as SEQ ID No. 7.
13. The process according to any one of the preceding clauses, wherein a conversion yield to a dicarboxylic acid is at least 60%.
14. The process according to any one of the preceding clauses, wherein the reaction step is stopped when there is 75% conversion of the aliphatic dinitrile to dicarboxylic acid.
15. The process according to any one of clauses 1 to 13, wherein the reacting step is continued until there is at least 80%.
16. The process according to any preceding clause wherein reacting step is carried out at a pH in the range of 6 to 8.
17. The process according to any one of the preceding clauses, wherein the dicarboxylic acid is chiral and the reaction yields an enantiomeric excess of 75% or more of one isomer of the dicarboxylic acid.
18. The process according to clause 17, wherein the enantiomeric excess of is 80% or more.
19. The process according to any one of the preceding clauses, wherein the reaction yields an enantiomeric excess of one isomer of mononitrile.
20. The process according to clause 19, wherein the enantiomeric excess is 70% or more.
21. The process according to any preceding clause wherein reacting step is carried out over a period of 6 days or less, and more preferably less than 4 days.
22. A process as stated in any preceding clause, wherein the source of the dinitrile contains more than one dinitrile compound.
23. A process as stated in any of clauses 1 to 21, wherein the source of the dinitrile contains one dinitrile compound.
24. A process as stated in any preceding clause, wherein the source of the dinitrile is a waste stream, preferably wherein the waste stream is a byproduct of the manufacture of adiponitrile (1,4-dicyanobutane).
25. A process as stated in any preceding clause, wherein the or each dinitrile is an aliphatic dinitrile.
26. A process as stated in any preceding clause, wherein the or each aliphatic dinitrile contains from 5 to 10 carbon atoms, and preferably contains 5 or 6 carbon atoms.
27. The process according to any one of the preceding clauses, wherein the dinitrile is 2-methylglutaronitrile and the dicarboxylic acid produced is 2-methylglutaric acid and / or wherein the dinitrile is 2-ethylsuccinonitrile and the dicarboxylic acid produced is 2-ethylsuccinic acid.
28. A process as stated in any of clauses 1 to 24, wherein the dinitrile is an aromatic dinitrile.
29. A process as stated in any of clauses 1 to 24 and 28, wherein the or each aromatic dinitrile contains from 6 to 10 carbon atoms, and preferably contains 6, 7, or 8 carbon atoms.
30. A process for producing diesters comprising the steps of:
   a. Producing a dicarboxylic acid in accordance with any one of the preceding clauses; and
   b. Converting the dicarboxylic acid to a diester by reaction with one or more alcohols, wherein the or each alcohol contains from 1 to 20 carbon atoms.
31. Use of a nitrilase in the production carboxylic acids and/or dibasic esters, wherein the nitrilase comprises a CWE motif and, wherein the nitrilase when tested using the OPA Nitrilase Assay with 2-methylglutaronitrile as substrate has an % total hydrolysis of at least 45% or when tested using the OPA Nitrilase Assay with 4-cyanopentanoic acid as substrate has an % total hydrolysis of at least 15%.
32. Use of a nitrilase in accordance with clause 31, wherein the nitrilase is a variant of SEQ ID No.1 and has an amino acid sequence which when aligned to SEQ ID No. 1 has a modification at one or more of Val 90, Leu 131 and Thr 243.
33. Use of a nitrilase in accordance with clause 32, wherein the modification at Val 90 is V90A; the modification at Leu 131 is L131I, L131V, L131Y or L131M; and/or the modification at Thr 243 is T243A.
34. Use of a nitrilase in accordance with any one of clauses 31 to 33, wherein the nitrilase has at least 90% sequence identity to SEQ ID No. 1.
35. Use of a nitrilase in accordance with any one of clauses 31 to 34, wherein the nitrilase has at least 90% sequence identity with a wild-type nitrilase from a Bradyrhizobium or Nitratireductor species.
36. Use of a nitrilase in accordance with any one of clauses 31 to 35, wherein the nitrilase has an amino acid sequence which has at least 90% sequence identity t any one of: SEQ ID No. 2, SEQ ID No. 3 and SEQ ID No. 5.
37. Use of a nitrilase in accordance with any one of clauses 31 to 36, wherein the nitrilase is immobilised.
38. Use of a nitrilase in accordance with any one of clauses 31 to 37, wherein the nitrilase is used in combination with an amidase.
39. Use of a nitrilase in accordance with clause 38, wherein the amidase is capable of reacting with 4-CPAmide.
40. Use of a nitrilase in accordance with clause 38 or clause 39, wherein the amidase is an aliphatic amidase (E.C. 3.5.1.4) which is a member of Pfam PF01425.
41. Use of a nitrilase in accordance with any one of clauses 36 to 38, wherein the amidase comprises a sequence set forth as SEQ ID No. 7.
42. Use of a nitrilase in accordance with any one of clauses 31 to 41, wherein a conversion yield to a dicarboxylic acid is at least 60%.
43. An isolated polypeptide having nitrilase activity, which comprises an amino acid sequence which when aligned to SEQ ID No. 1 has a modification at one or more of: Val 90, Leu 131 and/or Thr 243 and wherein:
   a. the amino acid sequence has at least 90% sequence identity to SEQ ID NO. 1; or
   b. the amino acid sequence binds under stringent conditions to the complement of SEQ ID No. 1.
44. The amino acid sequence according to clause 42, wherein the modification at Val 90 is V90A; the modification at Leu 131 is L131I, L131V, L131Y or L131M; and/or the modification at Thr 243 is T243A.
45. The amino acid sequence according to clause 43 or clause 44, wherein the nitrilase when tested using the OPA Nitrilase Assay with 2-methylglutaronitrile as substrate has an % total hydrolysis of at least 45% or when tested using the OPA Nitrilase Assay with 4-cyanopentanoic acid as substrate has an % total hydrolysis of at least 15%.
46. A nucleic acid encoding an amino acid in accordance with any of clauses 43 to 45.

## Claims

1. A process for the production of a dicarboxylic acid comprising the step of:
i) reacting a composition comprising one or more aliphatic dinitriles wherein the or each aliphatic dinitrile contains from 2 to 20 carbon atoms or one or more aromatic dinitriles wherein the or each aromatic dinitrile contains from 6 to 20 carbon atoms with a nitrilase to produce a dicarboxylic acid, wherein the nitrilase comprises a CWE motif and, wherein the nitrilase when tested using the OPA Nitrilase Assay with 2-methylglutaronitrile as substrate has an % total hydrolysis of at least 45% or when tested using the OPA Nitrilase Assay with 4-CPA as substrate has an % total hydrolysis of at least 15%.

2. The process according to claim 1 wherein the nitrilase is a variant of SEQ ID No.1 and has an amino acid sequence which when aligned to SEQ ID No. 1 has a modification at Val 90, Leu 131 and/or Thr243.

3. The process according to claim 1 or claim 2, wherein the nitrilase has at least 90% sequence identity to SEQ ID No. 1

4. The process according to any preceding claim wherein the reaction is carried out at a temperature of 40-45°C.

5. The process according to any preceding claim, wherein the nitrilase has an amino acid sequence which has at least 90% sequence identity to any one of: SEQ ID No. 2, SEQ ID No. 3 and SEQ ID No. 5.

6. The process according to any preceding claim, wherein the nitrilase is immobilised.

7. The process according to any one of the preceding claims, wherein an amidase is added prior, simultaneously or subsequent to reaction step i).

8. The process according to any one of the preceding claims, wherein the reaction step is stopped when there is 75% conversion of the aliphatic dinitrile to dicarboxylic acid.

9. The process according to any preceding claim wherein reacting step is carried out at a pH in the range of 6 to 8.

10. The process according to any one of the preceding claims, wherein the reaction yields an enantiomeric excess of one isomer of mononitrile, preferably wherein the enantiomeric excess is 70% or more.

11. The process according to any preceding claim wherein reacting step is carried out over a period of 6 days or less, and more preferably less than 4 days.

12. A process as claimed in any preceding claim, wherein the source of the dinitrile is a waste stream, preferably wherein the waste stream is a byproduct of the manufacture of adiponitrile (1,4-dicyanobutane).

13. A process as claimed in any preceding claim, wherein the or each aliphatic dinitrile contains from 5 to 10 carbon atoms, and preferably contains 5 or 6 carbon atoms, and more preferably wherein the dinitrile is 2-methylglutaronitrile and the dicarboxylic acid produced is 2-methylglutaric acid and / or wherein the dinitrile is 2-ethylsuccinonitrile and the dicarboxylic acid produced is 2-ethylsuccinic acid.

14. A process for producing diesters comprising the steps of:
a. Producing a dicarboxylic acid in accordance with any one of the preceding claims; and
b. Converting the dicarboxylic acid to a diester by reaction with one or more alcohols, wherein the or each alcohol contains from 1 to 20 carbon atoms.

15. Use of a nitrilase in the production carboxylic acids and/or dibasic esters, wherein the nitrilase comprises a CWE motif and, wherein the nitrilase when tested using the OPA Nitrilase Assay with 2-methylglutaronitrile as substrate has an % total hydrolysis of at least 45% or when tested using the OPA Nitrilase Assay with 4-cyanopentanoic acid as substrate has an % total hydrolysis of at least 15%.

16. An isolated polypeptide having nitrilase activity, which comprises an amino acid sequence which when aligned to SEQ ID No. 1 has a modification at one or more of: Val 90, Leu 131 and/or Thr 243 and wherein:
a. the amino acid sequence has at least 90% sequence identity to SEQ ID NO. 1; or
b. the amino acid sequence binds under stringent conditions to the complement of SEQ ID No. 1.

17. A nucleic acid encoding an amino acid in accordance with claim 16.
